(19)

Europäisches
Patentamt
European
Patent Office
Office européen
des brevets

(11) **EP 1 950 774 A2**

(12) # EUROPEAN PATENT APPLICATION

(43) Date of publication:
**30.07.2008 Bulletin 2008/31**

(51) Int Cl.:
*H01F 27/40* (2006.01)

(21) Application number: **07466025.9**

(22) Date of filing: **29.11.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK RS**<br><br>(30) Priority: **01.12.2006 CZ 20060755** | (71) Applicant: **Altmann, Josef**<br>**344 01 Domazlice (CZ)**<br><br>(72) Inventor: **Altmann, Josef**<br>**344 01 Domazlice (CZ)**<br><br>(74) Representative: **Langrova, Irena**<br>**Skretova 48**<br>**301 00 Plzen (CZ)** |

(54) **Quantitative measurement of production and consumption of gases in power transformers and device**

(57) The design relates to the process of quantitative measurement of generation or consumption of gases, particularly operational measurement of generation or consumption of gases in power transformers and device suitable for implementation of such a method.

The whole process of quantitative measurement in carried out in two steps. At first, the rate of flow of oil is examined as the oil is considered the carrier of all gases between the tank (10) of the transformer (1) and the conserver (11) implemented in the form of a dynamic change in calibration gas concentration within the oil filling (12) of the tank (10) of the transformer (1). Regarding the fact that the known volume of the oil filling (12) of the tank (10) of the transformer (1) is utilized as a well-definable capacity enabling the required dynamic change to occur in it.

The required change of calibration gas concentration within the oil filling (12) can also be achieved by two basic methods of the procedure:
If nitrogen N is utilized as the calibration gas, always presented in the oil filling, the oil filling (12) is vacuum treated, and subsequently the time interval based on a process of a natural reverse saturation of oil filling (12) is measured by nitrogen from the surrounding air environment.

The second procedure employs the method of compressive saturation of oil filling of the transformer implemented by the calibration gas, which is not contained in such oil filling under standard circumstances. The calibration gas is forced to supply the oil filling of the conserver (11) and consequently the growth of its concentration is measured in the oil filling (12) of the tank (10) of the transformer (1).

From the results of measured calibration gas concentration gradient in the oil filling (12) and values of calibration gas concentrations both in oil filling (12) of the tank (10) and in the oil filling of the conserver (11) is defined the volume flow rate between both tanks (10).

By means of known values of oil rate of flow determined between the tank (10) of the transformer (1) and the conserver (11) and the time sequence of measured concentrations of all monitored gases examined by the DGA analyser (3) in both tanks (10), flows per all monitored gases corresponding with the generation or consumption of such gases in the tank (10) of the transformer (1) are defined in the quantitative way.

Fig. 2

**Description**

The Technology Area

The invention relates to the method applied to quantitative measurement of the production or consumption of gases and device for implementation of such a process that is particularly suitable for quantitative measurement of production or consumption of gases in power transformers and other high voltage device saturated with transformer oil under an ordinary operation.

The State of Technology

Current measurement and identification of deficiencies related to the gas production or consumption in power transformers is implemented by means of so-called DGA method (Diluted Gas Analysis Method). This method utilizes the fact that gases either generated in the transformer or transported into it from air environment dissolve totally in its oil filling in ordinary circumstances. If we are interested in detecting whether the given gas is present in the transformer and in what concentration, it is sufficient to take a sample of oil gained from oil filling of the specific machine, thereupon displace the requested gas together with other gases and feed the gas mixture into the DGA analyser. The analyser output is relatively a high-accuracy measurement of the value of selected gases contained in the sampled oil.

Thereafter, this step is followed by a diagnostic procedure, which identifies gas sources from absolute values of measured gases and from their proportional representation, and based on the given gas concentration in oil, determines the scope of defect in the transformer, subsequently.

However, in practice it displays that such a defect diagnosis, i.e. sources of decomposition gases and gases generated in the process of oil oxidative ageing and cellulose materials in the transformer, represents serious deficiencies.

Elementary deficiency of the current diagnosis of transformer defects or intensity of its oxidative ageing appears to be an improper specification and perception of the very motion of gases in oil transformer filling.

It is possible to identify this motion for most of gases as a simple hydraulic analogy. Virtually, oil filling of the transformer tank can be replaced by the tank capturing gas leaking in from a particular defect. Concurrently, this gas also abandons this tank (in fact, the gas produced by the flaw is diluted in oil filling and simultaneously, is the diluted oil gas drifted away from the transformer tank by the oil flow to the conservator, and escapes to the air environment by the diffusion).

Gas concentration within the oil filling of the transformer is not merely defined by inlet intensity or the gas outflow (i.e. by the amount of the gas produced or consumed by the transformer defect), but by the intensity of the outflow, as well. Otherwise, by the amount of the gas transported out of the tank or flowed into it (amount of the gas escaping from the transformer tank and releasing to the air environment or flowing from the air environment into the tank, vice versa).

There is an apparent fundamental flaw of the current diagnostic procedure accepted from this analogy. Whereas, the content of the monitored gas in oil could be detected by the DGA analyser in a relatively very high accuracy, it does not enable to implement the existing measurement under approximately stabilized conditions and in no simple way detect the amount of gas flowed in and discharged from the transformer tank out of this measured value, nor what has caused a potential accrual or loss of this observed value. Therefore, the flaw size, which produces such a gas (or consumes), does not allow to explicitly and unfailingly define nor quantify, whether the flaw reduces or extends.

An extreme gas concentration in the oil filling of the transformer tank may also be caused by a high gas inlet from the flaw into the oil filling of the transformer as well as a reduced gas exhaust from the transformer tank into the environment.

In other words - the measurement of the gas concentration itself contained in oil does not provide us any relevant information nor is able to give any, in principle, expressed in standard volume or mass units, both about the flaw size in the transformer and its potential change. The same is true about oil oxidative ageing intensity and cellulose that we intend to find out in the transformer.

This deficiency in existing diagnosis is evaded by this common practice, the outflow of gases from the transformer tank to the environment is definitely considered a constant, and the diagnostic deduction is based on the comparison of existing and prior measured values of individual gases in oil. Unless the gas contained in oil rises, the current diagnosis explicitly associates such a growth with a higher production of the gas in the transformer tank, and vice versa in case of the decline, it is assumed that the gas production declined by the flaw.

Such an assumption is fallacious, because the gas vent from the transformer tank is not constant, but arranged mostly by the oil variable flow rate between the transformer tank and the conserver. The oil flow is controlled by the temperature time variation of the transformer oil filling (the flow rate specified between the transformer tank and the conserver is determined on the basis of oil filling expansion) and by the temperature difference of oil filling and the environment, which affects the constant oil flow identified between the transformer tank and the conserver in the frame of the thermo-siphon effect.

If the transformer temperature changes, its load modifies or the temperature of the environment changes, etc., it causes an immediate change of the oil flow rate between the transformer tank and the conserver followed by modified concentration of all gases subsequently diluted in oil filling of the transformer tank, though, own production of the gas does not change.

A relatively negligible flaw in the transformer may cause an extensive growth of the gas concentration at a low level of the gas outflow from the transformer tank into the machine tank, and will be diagnosed as an extensive flaw - vice versa, a relatively negligible flaw can cause a small rise of gas concentration at an intensive gas outflow from this tank, and consequently will be interpreted as a negligible one.

Moreover, the current DGA does not enable to quantify nor the oil flow rate between the machine tank and the conserver, in principle, and the amount of gas that is between both oil tanks transported.

The measurement interpretation conceived as a standard inevitably produces a wide range of more or less anomalous conclusions of the status of the monitored transformer.

Therefore, this diagnostic method has at least three entirely fundamental shortages:

  ➢ Does not respect physical reality of the measured system and when implementing the measurement it employs a categorical suggestion of a constant outflow / supply of gases from the system, which is generally imperfect, and moreover such an a priori suggestion is not verified by any quantitative method

  ➢ Is not capable of reviewing the flaw size in a standardized, i.e. quantitative measurement, providing us a truly relevant information in the way to state the amount of the gas produced by the flaw in a time period

  ➢ Is not explicitly capable of a differentiation, whether the measured growth or decline of the specific gas content is caused by the growth or reduction of the flaw, or vice versa, by reducing or extending of the discharge / gas supply from / into the transformer tank.

The Nature of Invention

Declared deficiencies are essentially limited by the method of a quantitative measurement of the production or consumption of gases and a device to implement such a procedure based upon the invention employing the DGA as a varied physical approach to diagnose power transformers defects.

New method of the quantitative measurement is based on the following suggestions:

  □ Oil filling of the transformer tank whose capacity could be accurately defined is employed as a measuring capacity enabling a precise physical and mathematical description of its dynamic behaviour

  o Actual oil flow rate specified between the conserver and the transformer tank allows to be measured in the following way - first, the oil filling of the transformer tank will be either released from or saturated by the calibration gas and subsequently we are able to monitor the growth (or decline) of the gas content dissolved both in the oil filling of the transformer tank and in the conserver, as well.

  □ Flows of all gases into / from the transformer tank are able to be defined by:

    o Known oil flow rate between the transformer tank and the conserver

    o Measuring the concentration of all relevant gases participating both in the transformer tank and in the conserver

    o Measuring the accrual gradient (or decline) of participating gases in this tank

To achieve a sufficient dynamic response in the calibration gas concentration and thus the requested measurement accuracy it is applied:

  ➢ Vacuum treatment of the transformer tank oil filling - first, the oil filling is vacuum treated, i.e. stripped of the calibration gas (all other gases, as well) dissolved in it and upon disconnection of the vacuum gas separator, the process of own dynamic response measurement is initiated, i.e. a saturation of the calibration gas in the examined system (usually by nitrogen) and by other gases from the conserver

  ➢ Supply of a suitable calibration gas into the conserver to achieve a fast growth of the calibration gas content in the conserver oil filling and consequently to achieve a gradual increase in the gas concentration within the oil filling of the transformer tank.

Nevertheless, the calibration gas (K) must behave in the transformer as an inert gas (neither is allowed to be produced nor consumed in this system) and thus, the change dynamics of its concentration in the transformer tank having the oil capacity of $V_n$, could be characterized by a simple differential equation:

$$(1) \quad V_n \cdot dC_{K,n} / dt = v_o \cdot (C_{K,kon} - C_{K,n})$$

Bellow explaining:

$V_n$          Known oil capacity in transformer tank
$dC_{K,n} / dt$     Saturation gradient of calibration gas (K) in transformer tank oil filling
$v_o$          Volume oil flow rate between conserver and machine tank
$C_{K,kon}$        Calibration gas content in oil conserver filling
$C_{K,n}$         Calibration gas content in transformer tank oil filling
dt          Time accrual

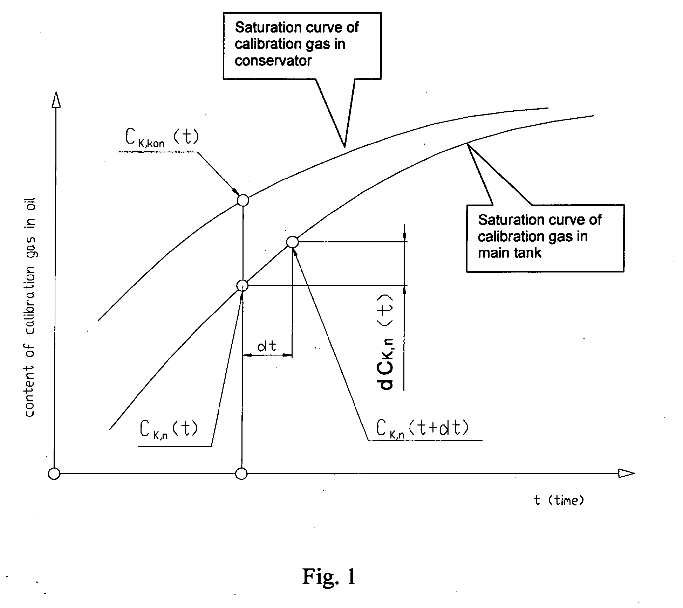

The simple mathematic model enables to define the saturation gradient $dC_{K,n} / dt$ of the calibration gas in the transformer tank based on a measured change of the calibration gas concentration. Both in the tank $C_{K,n}$ and in the conserver $C_{K,kon}$ (See Figure 1 - depicting time variations of the calibration gas concentration measured in the transformer tank as well as in its conserver).

Oil volume flow between the tank and the conserver can be calculated simply by the bellow mentioned mathematical expression:

$$(2) \quad v_o = V_n \cdot (dC_{K,n} / dt) \ / (C_{K,kon} - C_{K,n})$$

In other words, we have realized what oil volume flow $v_o$ is necessary:

☐ in known capacity, i.e. oil volume in the transformer tank $V_n$

☐ at measured concentration difference of the calibration content $C_{K,kon} - C_{K,n}$ between the conserver and the transformer tank

☐ measured time variation of the calibration gas $dC_{K,n} / dt$ occurred in the transformer tank

Subsequent definition of the **production** of the gas X by the flaw in the transformer tank is actually easy, because each single gas X behaves in oil filling of the transformer tank as though it would be the only one in oil filling, thus the change dynamics of the gas concentration could be expressed by a differential equation:

$$(3) \quad V_n \cdot dC_{X,n} / dt = v_o \cdot (C_{X,kon} - C_{X,n}) + v_X$$

Therefore, there is a known development of concentrations in time identified for the given gas X both in the tank and in conserver, we are repeatedly able to define its gradient $dC_{X,n} / dt$ as well as the gas flow $v_X$ because of the flaw generating the gas X, thus it is stipulated by the following mathematical expression:

$$(4) \quad v_X = V_n \cdot dC_{X,n} / dt - v_o \cdot (C_{X,kon} - C_{X,n})$$

Bellow explaining:

$v_X$            Volume flow of the gas X generated in the transformer tank

$dC_{X,n} / dt$ — Accrual gradient of the gas concentration X in the transformer tank

$v_o$ — Recognized oil volume flow circulating through the conserver and the machine tank

$C_{X,kon}$ — Content of the gas X measured in oil filling of the conserver

$C_{X,n}$ — Content of the gas X measured in oil filling of the transformer tank

It is possible to recognize even the consumption of the gas in the transformer tank in the same and simple way. Regarding the transformer oxidative aging, we are merely interested in oxygen consumption (O2), thus this process dynamics is to be interpreted according to the following mathematical expression (5):

$$(5) \quad V_n \cdot dC_{O2,n} / dt = v_o \cdot (C_{O2,kon} - C_{O2,n}) - v_{O2}$$

Therefore, we know the oil flow $v_o$ passing through the transformer tank and the conserver, the volume oxygen consumption in the transformer tank is given by the expression:

$$(6) \quad v_{O2} = v_o \cdot (C_{O2,kon} - C_{O2,n}) - V_n \, dC_{O2,n} / dt$$

$V_{O2}$ — Volume oxygen flow consumed in the transformer tank

$dC_{O2,n} / dt$ — Accrual gradient of the oxygen concentration in the transformer tank

$v_o$ — Volume oil flow circulating between the conserver and the machine tank

$C_{O2,kon}$ — Oxygen content measured in oil filling of the conserver

$C_{O2,n}$ — Content of the oxygen gas measured in oil filling of the transformer tank

The advantage of this quantitative measurement of the production or consumption of gases based on the invention is particularly the fact that such a measurement method enables to provide us a relevant quantitative idea on the flaw size existing in the transformer.

Another advantage seen in this measurement method is a possibility to perform a reverse measurement calibration acquired because of a standard methodology or a detection and elimination of potential devious diagnostic conclusions executed according to this methodology.

Another cardinal advantage of such a method is the fact that this procedure can be carried out at all times in ordinary transformer operation and without any larger technological adjustments.

Brief Description of the Drawings

Fig. 1 - Change Dynamics of the calibration gas content dissolved in oil filling of the transformer tank and the conserver, where the curve n depicts a measured content of the calibration gas in the oil filling of the transformer tank and the curve k depicts a measured content of the calibration gas dissolved in oil filling of the conserver. Vertical axis - Content of Calibration Gas in Oil

Fig. 2 - Device, through which the measurement is carried out based on the vacuum treatment of the oil filling of the transformer tank.

Fig. 3 - Device, through which the measurement is implemented based on a single controlled saturation of the oil filling of the transformer tank by the calibration gas.

The Examples of the Invention

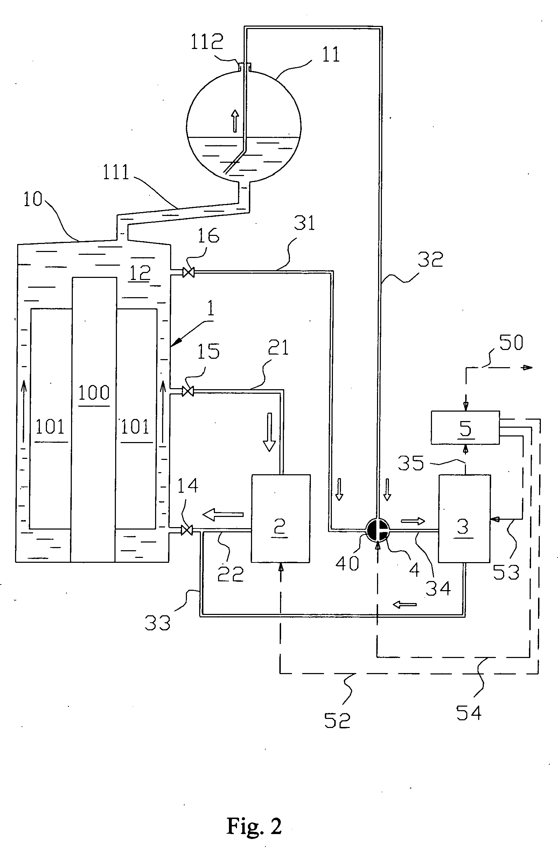

The first example of a practical implementation of the process dealing with quantitative measurement of the generation or consumption of gases related to the patent, is displayed in Fig. 2, depicting the device, through which is the measurement implemented based on a vacuum treatment of the oil filling of the transformer tank.

Device consist of the vacuum separator 2, DGA analyser 3, hydraulic switch 4 and the Process Control Computer 5 connected to the measured transformer 1.

Herein, the transformer 1 consists of the tank 10 with integrated active part, such as magnetic circuit 100 and winding 101 nested in oil filling of the tank 12 attached to the conserver 11 by gravitation pipe lines 111 from the above towards down. The upper part of the piping is either equipped with outlets of air pipes, not drawn in the figure, interconnecting the conserver 11 with ambient atmosphere, or either there is a plug 112 positioned in its upper part. Simultaneously, the upper bleeding valve 16 is built into the side part of the tank 10 of the transformer 1, the medium bleeding valve 15 and the bottom bleeding valve 14. Actual hydraulic interconnection of the transformer 1 with the vacuum separator 2 is carried out either by induction piping 21 flowing from the medium bleeding valve 15 and emptying into the upper part of the vacuum separator 2, or by a pressure conduit 22 coming from the left side of the vacuum separator 2. It is attached to the bottom bleeding valve 14. The hydraulic interconnection of the transformer 1 and the DGA analyser 4 is carried out either by the first take-off pipe 31, attached to the upper bleeding valve 16, and by its second end attached to the hydraulic switch 4. In this case, its rotary switch 40 turns through 90° clockwise as compared to the position drawn in the Fig. 2 and interconnects the first take-off pipe 31, the binding pipe 34 with the DGA analyser 3, and both through the second take-off pipe 32 with its upper part emptying into the bottom part of the conserver 11. It comes through the plug 112 and the bottom part of this conduit leads into the hydraulic switch 4 from above. In this second case, its rotary switch 40 as drawn in the position in Fig. 2, interconnects the second take-off pipe 32 with the binding pipe 34 and the DGA analyser 3. While the DGA analyser 3 is interconnected with the return pipe 33 and oil filling of the tank 12 in such a way that the return pipe 33 empties into the pressure conduit 22 of the vacuum separator 2 in front the bottom bleeding valve 14, furthermore.

Electric connection of the vacuum separator 2, the DGA analyser 3, hydraulic switch 4 and the Process Control Computer 5 are within the scope of device assured by the set of electric interconnecting devices with respect to the patent.

The Process Control Computer 5 is connected to the DGA analyser 3 either by the transmission line 35, or by the first electric control inter-connector 53. Furthermore, the Process Control Computer 5 is tied in the second electric control inter-connector 54 equipped with the hydraulic switch 4 and next tied in with the third electric control inter-connector 52 with the vacuum separator 2. Currently, the Process Control Computer 5 is also considered to be connected via the communication line 50 to the not drawn superior computer.

Activities of the device as related to the patent proceeds in two basic steps. First, the oil filling 12 of the tank 10 of the transformer 1 is in the first stage discharged of the calibration gas by the vacuum separator 2 as well as other gases dissolved in it. In the second phase, upon the vacuum separator 2 switch-off is the oil filling of the tank 10 of the transformer 1 spontaneously saturated again by the calibration gas (herein by nitrogen N) and other gases. The Process Control Computer enables to monitor, control and evaluate the whole process in terms of values given by the DGA analyser 3.

After the process of network hydraulic connection of the vacuum separator 2, the hydraulic switch 4 and the DGA analyser 3 to the transformer 1, and after the following electric interconnection among all mentioned components with the Process Control Computer 5, and in case of the connection established between the Process Control Computer 5 and the superior computer, the whole device is in terms of the patent including all inter-connection piping, vacuum treated on the basis of the instruction given by the Process Control Computer 5. In order to eliminate the possibility to import or impress air bubbles into the oil filling 12 of the tank 10 of the transformer 1 entirely, the vacuum separator 2 implements it.

Subsequently, the vacuum separator is gradually put into standard operation at which the oil from the oil filling 12 of the tank 10 of the transformer 1 flows through the medium bleeding valve 15 and through the induction piping 21 into the vacuum separator 2, where it is separated from the dissolved gases and the de-aerated oil received from the vacuum separator 2 is discharged via the pressure conduit 22 and the bottom bleeding valve 14 backwards to the oil filling 12 of the transformer 1.

Simultaneously, the second take-off pipe 32 absorbs the oil from the conserver 11; it flows via hydraulic switch 4 and the binding pipe 34 into the DGA analyser 3 where the concentration of the calibration gas and other monitored gases is measured. The oil from the DGA analyser 3 is pumped via the return pipe 33 into the pressure conduit 22, and together with the oil transported from the vacuum separator 2, it is imported backwards by the bottom bleeding valve 14 into the oil filling 12 of the transformer 1. Information on measured concentration of the calibration gas and other gases contained in the oil filling of the conserver 11 is imported from the DGA analyser 3 via the transmission line 35 into the Process Control Computer 5, and eventually also by the communication line 50 to a not drawn remote computer.

Nevertheless, the process control computer 5 modifies the position of its hydraulic switch 40 by a command issued by the second electric control inter-connector 54 to the hydraulic switch 4 at pre-programmed time intervals so that by alternative rotation of the hydraulic switch 40 in the clockwise and anti-clockwise direction. The oil filling 12 from the tank 10 of the transformer 1 flows in to the DGA analyser 3 in turns as well as the oil from the conserver 11.

Thereafter, the vacuum treatment of the oil filling 12 of the tank 10 of the transformer 1 continues for such a long time until the distinction between concentrations of calibration gas (hereinafter referred to nitrogen) and the oil filling 12 of the tank 10. The oil in the conserver 11, does not reach the value that would guarantee a required accuracy measurement in the following saturation stage.

Then, the vacuum separator 2 is switched off by the third electric control inter-connector 52 based on the command issued by the Process Control Computer 5, and the whole device enters another saturation phase in compliance with the patent. In this phase, the oil filling 12 of the transformer 1 is saturated by the calibration gas and other monitored gases.

The Process Control Computer 5 switches again the hydraulic switch 4 at selected time intervals in order to assure that the oil from the oil filling 12 of the tank 10 of the transformer 1 and the conserver 11 would be flowing in to the DGA analyser 3 in turns. All results received from all measurements are from the DGA analyser 3 transferred back to the Process Control Computer 5 by the transmission line 35.

The Process Control Computer 5 continually compares measured concentrations of the calibration gas both in the oil filling 12 and in the conserver 11 of the transformer 1. Simultaneously, it calculates the rate of flow between the conserver 11 and the tank 10 of the transformer 1, and thus ensures achieved accuracy of measurements per all gases monitored by their comparison together with the mathematic model. The achieved level of evaluation is via the communication line 50 also transmitted from the Process Control Computer 5 to the not drawn remote computer to be ready for a potential check.

In the instant of achieving the required accuracy of measurements, the process of examination itself is completed, and the Process Control Computer 5 automatically executes quantitative determination of flows per all measured gases in / from the oil filling 12 of the tank 10 of the transformer 1.

Unless the required accuracy is not achieved at the given time interval, the Process Control Computer 5 will refer such a fact to its superior workstation and will execute a new measurement, i.e. will re-vacuum treat the oil filling 12 of the transformer 1 and will execute a new calculation of flows per all examined gases.

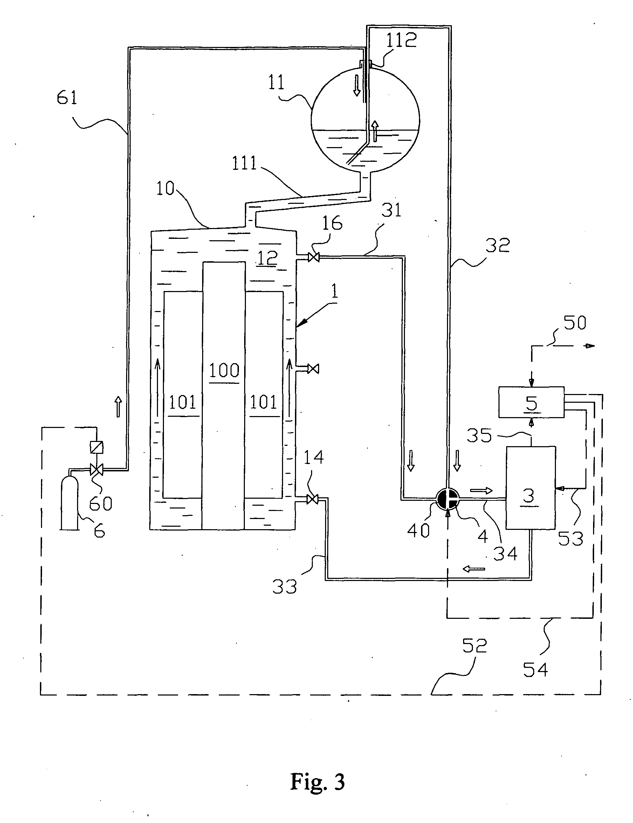

The second example of a practical implementation of quantitative measurement of generation or consumption of gases in terms of the patent is demonstrated in Fig. 3. It depicts device, through which the measurement based on a single controlled saturation of the oil filling into the tank 10 of the transformer 1 by the calibration gas, is implemented.

Exemplary implementation of the device is depicted in Fig. 3 consisting of the pressure cylinder 6 with compressed calibration gas, the DGA analyser 3, the hydraulic switch 4 and the Process Control Computer 5 attached to the examined transformer 1.

Hereto, the respective structure of examined power transformer 1 is identical to the first example of the practical implementation of device as determined by Fig. 2 with the only exception. It is represented by the inlet of the calibration gas conduit 61 into the upper part of the conserver 11.

Hydraulic interconnection of the transformer 1 with the DGA analyser 3 is implemented either by the first take-off line 31 attached onto the upper bleeding valve 16 and by its second end attached onto the hydraulic switch 4, whereby the rotary switch 40 is rotated clockwise in this case at 90° compared with the position designed in Fig. 2. It interconnects the first take-off line 31, binding pipe 34 with the DGA analyser 3, and partly by the second take-off line 32, whereby its upper part inlet leads into the bottom part of the conserver 11, passes through the plug 112 and the bottom part of this line is installed from its upper side into the hydraulic switch 4 having the rotary switch 40. In this case, in the position drawn in Fig. 2, interconnects the second take-off line 32, via the binding pipe 34 with the DGA analyser 3, whereas the DGA analyser 3 is attached to the return pipe 33 and via the bottom bleeding valve 14 with the oil filling 12 of the transformer 1, further on.

The interconnection between the pressure cylinder 6 and the transformer 1 consists of the gas pipeline 61 leading from the servo-operated valve 60 located on the pressure cylinder 6 and passes through the plug 112 and has the outlet situated above the oil level in the conserver 11.

Electric interconnection of the DGA analyser 3, hydraulic switch 4, Process Control Computer 5 and the servo-operated valve 60 is assured by a circuitry of electric interface equipment in the device based on the patent.

The Process Control Computer 5 is attached to the DGA analyser 3 either by transmission line 35 and either by the first electric control inter-connector 53. Hereinafter, the Process Control Computer 5 is connected through the second electric inter-connecter 54 with the hydraulic switch 4, and subsequently with the third electric inter-connector 52 to the servo-operated valve 60. Simultaneously, the Process Control Computer 5 is also attached by the communication line 50 to not drawn superior computer.

Activities of the second practical implementation related to the patent are carried out at any times under ordinary operation of the transformer 1 and as compared to the first practical example, there is no need to vacuum treat the oil filling 12 of the transformer 1.

Upon the command issued by the Process Control Computer 5 transmitted via the third electric inter-connector 52, the servo-operated valve 60 opens on the pressure cylinder 6 and the calibration gas starts flowing in from the pressure cylinder 6, gas conduit 61 situated above the oil level in the conserver 11. It diffuses to the oil filling of the conserver 11 under the conditions of moderated over-pressure, and consequently together with other gases is transported outside of the conserver 11 via a standardized binding line, which connects the inner space of the conserver with the air environment not drawn in Fig. 3.

The calibration gas concentration above the oil level in the conserver 11 gradually grows supported by this process and a part of the calibration gas dissolves in its oil filling. Based on the oil rate of flow in the binding line between the conserver 11 and the tank 10 the calibration gas concentration gradually grows within oil filling 12 of the transformer 1. The whole process is fully monitored, controlled and evaluated by the process control computer 5 in terms of values measured by the DGA analyser 3.

Information on examined calibration gas concentration and other gases contained in the oil filling of the conserver 11 is via the transmission line 35 implemented from the DGA analyser 3 into the Process Control Computer 5 and also via communication line 50 transmitted to the not drawn superior computer, eventually.

The Process Control Computer 5 modifies the position of its rotary switch 40 on the basis of pre-programmed time intervals by the command transmitted by the second electric control inter-connector 54 to the hydraulic switch 4 so that by some alternative rotation of the rotary switch 40 in the clockwise and anti-clockwise direction, the oil from oil filling 12 of the tank 10 of the transformer 1 and the conserver oil 11 flow in to the DGA analyser 3 in turns.

Thereafter, the vacuum treatment of oil filling 12 of the tank 10 of the transformer 1 continues for such a long time until the difference from concentrations between calibration gas and oil filling 12 of the tank 10 and oil in the conserver 11 does not reach the value assuring the required accuracy of the measurement.

The Process Control Computer 5 compares measured concentrations of the calibration gas both in the oil filling 12 and in the conserver 11 of the transformer 1, and simultaneously it calculates the oil rate of flow between the conserver 11 and the tank 10 of the transformer 1, and thus ensures achieved accuracy of measurements per all monitored gases. Measurement results and their evaluation are also transmitted from the Process Control Computer 5 via the communication line 50 to the not drawn remote computer to be ready for a potential check.

In the instant of achieving the required accuracy of measurements, the process of examination itself is completed, and the Process Control Computer 5 automatically executes quantitative determination of flows per all measured gases from / in the oil filling 12 of the tank 10 of the transformer 1. By switching, the servo-operated valve 60 off, it disconnects calibration gas supply to the conserver 11.

The calibration gas spontaneously escapes to the atmosphere and its concentration both in the oil filling 12 of the transformer 1, and in the conserver oil filling 11 gradually decreases up to the trace concentration established prior to the beginning of examination, consequently.

List of Reference Symbols

1       Transformer
10      Transformer Tank
100     Magnetic Circuit
101     Winding
11      Conserver
111     Gravitation Pipelines
112     Plug
12      Oil Filling
14      Bottom Bleeding Valve
15      Medium Bleeding Valve
16      Upper Bleeding Valve

2       Vacuum Separator
21      Induction Piping
22      Pressure Conduit

3       DGA Analyser
31      First Take-off Pipe
32      Second Take-off Pipe
34      Binding Pipe
33      Return Pipe

4       Hydraulic Switch
40      Rotary Switch

5       Process Control Computer
50      Communication Line
35      Transmission Line
53      First Electric Control Inter-Connector

52    Third Electric Control Inter-Connector
54    Second Electric Control Inter-Connector

6     Pressure Cylinder
60    Servo-operated Valve
61    Calibration Gas Conduit

**Claims**

1.  The process of quantitative measurement of generation or consumption of gases implemented in power transformers, **characterized by** the fact that the concentration of all relevant gases is continually measured by the DGA analyser (3) both in oil filling (12) of the tank (10) of the transformer (1) and in the oil filling (12) of the conserver (11) as well as known volume of oil filling (12) of the tank (10) of the transformer (1) that is applied in the form of a measuring capacity regarding the fact that the oil rate of flow between the tank (10) of the transformer (1) and the conserver (11) is measured through the dynamic response procedure reacting to the change of the calibration gas inlet or outlet from / in the oil filling (12) of the tank (10) of the transformer (1).

2.  The process of quantitative measurement according to the Claim 1, **characterized by** the fact that the required dynamic change of the calibration gas concentration already contained in oil is reached by the fact that all gases dissolved in the oil filling (12) of the tank (10) of the transformer (1) are removed, initially.

3.  The process of quantitative measurement according to the Claim 1, **characterized by** the fact that the required dynamic change of the calibration gas concentration in oil filling (12) of the tank (10) of the transformer (1), is achieved by the fully controlled calibration gas supply to the oil filling (12) of the transformer (1).

4.  The process of quantitative measurement according to the Claims 1, 2 and 3, **characterized by** the fact that both the measured time variation of the calibration gas concentration in known volume of the oil filling (12) of the tank (10) of the transformer (1) and the measured variation of calibration gas concentrations in oil filling (12) of the tank (10) of the transformer (1) and the conserver (11), are used to define the rate of flow between the oil filling (12) of the tank (10) of the transformer (1) and the conserver (11).

5.  The process of quantitative measurement according to the Claims 1 to 4, **characterized by** the fact that through the recognized oil rate of flow between the tank (10) of the transformer (1) and the conserver (11) and the time interval of measured concentrations of all monitored gases examined by the DGA analyser (3) in both tanks (10) defines flows of all monitored gases in the quantitative way corresponding to the generation or consumption of these gases in the tank (10) of the transformer (1).

6.  Device to implement the process of the quantitative measurement of the generation or consumption of gases contained in the vacuum separator, DGA analyser, hydraulic switch, Process Control Computer, system of tubing and electric supply conduit, **characterized by** the fact that the vacuum separator (2) is partly attached by the induction pipes (21), partly by the pressure conduit (22) to the tank (10) of the transformer (1) and partly attached by the third electric control inter-connector (52) also to the Process Control Computer (5) whereas the DGA analyser (3) is attached through the binding pipe (35) to the hydraulic switch (4) and by the return pipe (33) to the pressure conduit (22) and via the first electric control inter-connector (53) and the transmission line (35) to the process control computer (5), while the hydraulic switch (4) is partly interconnected by the first take-off pipe (31) to the tank (10) of the transformer (1) and by the second take-off pipe (32) to the bottom part of the conserver (11) in turns in dependence on the position of the rotary switch (40) and concurrently it is also attached via the second electric control inter-connector (54) to the process control computer (5), which is via the communication line (50) attached to the superior computer.

7.  Device to implement the process of the quantitative measurement of the generation or consumption of gases consisting of the pressure cylinder with calibration gas, the DGA analyser, hydraulic switch, process control computer, system of tubing and electric supply, **characterized by** the fact that the pressure cylinder (6) is attached via the calibration gas conduit (61) with the inner space of the conserver (11) whereas, the DGA analyser (3) is attached through the binding pipe (35) to the hydraulic switch (4) and via the return pipe (33) interconnected with the oil filling (12) as well as via the first electric control inter-connector (53) and the transmission line (35) to the process control computer (5) while the hydraulic switch (4) is interconnected hydraulically via the first take-off pipe (31) to the tank (10) of the transformer (1) and via the second take-off pipe (32) to the bottom part of the conserver (11) in turns and depending on the position of the rotary switch (40), concurrently it is also interconnected via the second electric control inter-connector (54) to the process control computer (5), which is via the communication line (50) attached to the process control computer.

Fig. 1

Fig. 2

**Fig. 3**